(19) Europäisches Patentamt
European Patent Office
Office européen des brevets

(11) Publication number: 0 429 436 A2

# (12) EUROPEAN PATENT APPLICATION

(21) Application number: 91200118.7

(22) Date of filing: 04.12.86

(51) Int. Cl.5: C07D 498/18, A61K 31/395, //(C07D498/18,311:00,273:00, 221:00)

This application was filed on 22.01.1990 as a divisional application to the application mentioned under INID code 60.

(30) Priority: 06.12.85 US 806152

(43) Date of publication of application: 29.05.91 Bulletin 91/22

(60) Publication number of the earlier application in accordance with Art.76 EPC: 0 227 355

(84) Designated Contracting States: AT BE CH DE ES FR GR IT LI LU NL SE

(71) Applicant: THE UNIVERSITY OF KANSAS
Office of Research, Graduate Studies and Public Service, 226 Strong Hall
Lawrence, Kansas 66045-2300(US)

(72) Inventor: Stella, Valentino John
777 Sunset Drive
Lawrence, Kansas(US)
Inventor: Kennedy, Paul Edwin
5101 River Road
Bethesda, Maryland(US)

(74) Representative: Wileman, David Francis Dr. et al
c/o Wyeth Laboratories Huntercombe Lane South
Taplow Maidenhead Berkshire SL6 OPH(GB)

## (54) Prodrugs of rapamycin.

(57) Water soluble prodrugs of rapamycin are disclosed which are useful as components in injectable pharmaceutical formulations for the treatment of tumors in mammals. Said prodrugs are rapamycin derivatives or salts thereof mono substituted by an acylamino substituent of formula

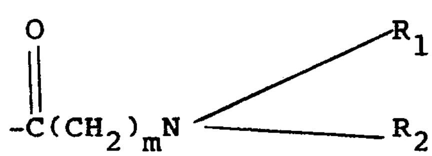

wherein m is an integer from 1 to 3 and $R_1$ and $R_2$ are each hydrogen or an alkyl radical having from 1 to 3 carbon atoms or $R_1$ and $R_2$ together with the nitrogen atom to which they are attached form a saturated heterocyclic ring having 4-5 carbon atoms.

EP 0 429 436 A2

## PRODRUGS OF RAPAMYCIN

This invention relates to water soluble prodrugs of rapamycin and in particular to certain .acylamino derivatives of rapamycin such as, for example, the glycinate prodrugs of rapamycin, the propionate of rapamycin and the pyrrolidino butyrate prodrugs of rapamycin.

Rapamycin is a known compound described and claimed in United States Letters Patent Nos. 3,929,992, issued December 30, 1975, and 3,993,749 issued November 23, 1976. Moreover, certain of its acyl derivatives are disclosed and claimed in United States Letters Patent No. 4,316,885, issued February 23,1982.

Rapamycin has been disclosed and claimed as useful in the treatment of tumors in Belgian Patent No. 877,700. Rapamycin is, however, only very slightly soluble in water, i.e. 20 micrograms per milliliter, and special injectable formulations have been developed for administration to patients, such as those described and claimed in European Patent Number EP 41,795. These formulations are not altogether satisfactory for a number of reasons including toxicity of the carrier. Accordingly, there is a need in the art for a rapamycin derivative or prodrug which is relatively soluble in water so as to form a safe injectable solution and which is as effective as rapamycin in the treatment of tumors.

It has now been found that water soluble prodrugs of rapamycin can be synthesized which decompose into products including rapamycin in the presence of human plasma and animal tissue homogenates. Such prodrugs of rapamycin provide a component of a valuable pharmaceutical injectable composition for the treatment of tumor in humans.

The water soluble prodrugs of this invention comprise mono-substituted derivatives at position 28 and disubstituted derivatives at positions 28 and 43 of the rapamycin structure. The assignments are based on a structural elucidation published by Findlay et al in Can. J. of Chem. 58, 579 (1980). This structure is shown below:

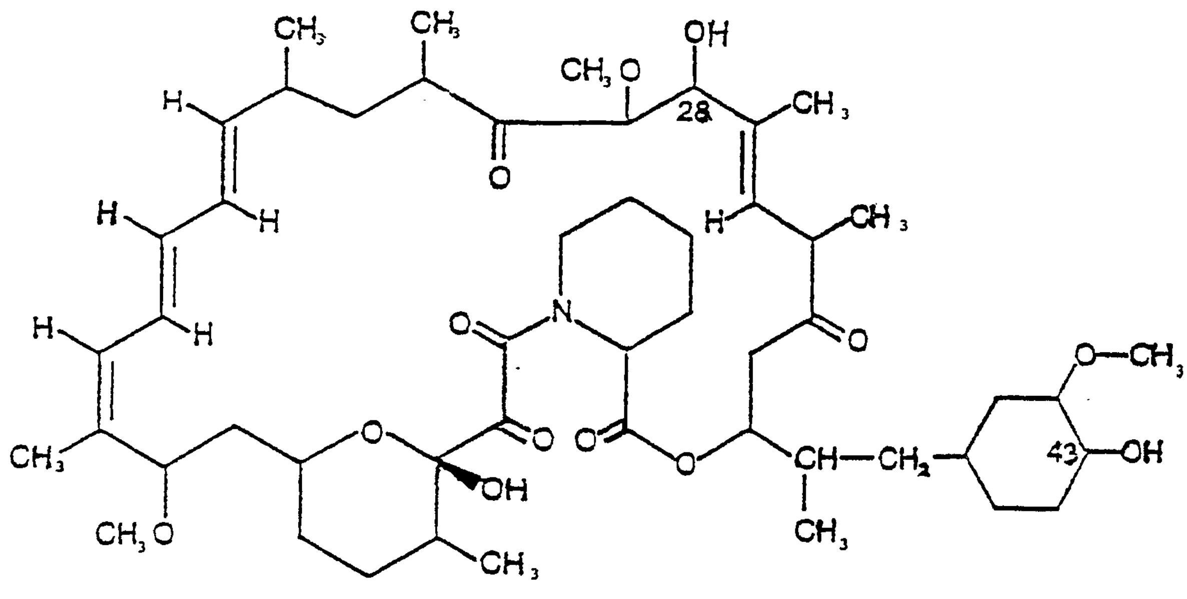

Rapamycin

The mono-substituted derivatives include those having a substituent at position 28 of the rapamycin structure having the following configuration.

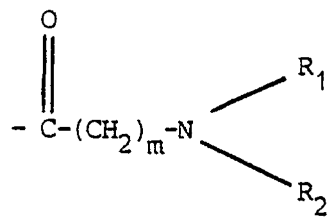

wherein m is an integer from 1 to 3, wherein $R_1$ and $R_2$ are each hydrogen or an alkyl radical having from one to three carbon atoms or wherein $R_1$ and $R_2$ together with the nitrogen atom to which they are attached form a saturated heterocyclic ring having four to five carbon atoms.
Compounds in which $R_1$ and $R_2$ are each an alkyl radical of one to three carbon atoms are preferred.
Examples of $R_1$ and $R_2$ are methyl and ethyl. Examples of m are 1 and 2.

The di-substituted derivatives include those having substituents at both positions 28 and 43 of the rapamycin structure having the same configuration as the substituent for the mono-substituted derivative.

This invention also provides processes for preparing the mono-and di-substituted water soluble prodrugs of rapamycin.

Accordingly this invention provides a process for preparing a water-soluble rapamycin derivative mono-substituted at position 28 or disubstituted at positions 28 and 43 by an acylamino substituent having the configuration

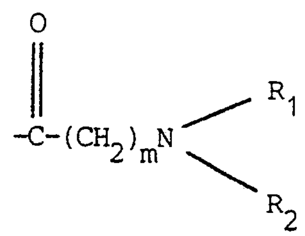

wherein m, $R_1$ and $R_2$ are as defined above which comprises acylating rapamycin with an acylating agent containing the said acylamino substituent having the configuration

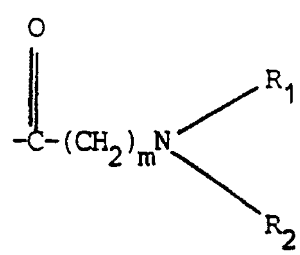

The acylation may be carried out by standard acylation procedures which preferably foster neutral conditions. The acylating agent may be the acid, the acid halide (i.e. the chloride or bromide), the acid annhydride or an activated ester of said acylamino substituent. The acid form of the acylamino substituent is preferred as an acylating agent in the presence of a suitable coupling agent. The particular coupling agent may be most effective in the presence of a catalyst and/or an acid scavenger. Examples of preferred coupling agents are N,N'-dicyclohexylcarbodiimide, 1,1'-carbonyldiimidazole, diethylazodicarboxylate, 2,2'-dithiopyridine annd N,N-diisopropylcarbodiimide. Diethylazodicarboxylate and 2,2'-dithiopyridine require the use of a catalyst such as triphenylphosphine. With these two coupling agents and triphenylphosphine as a catalyst a non-chlorinated solvent, such as an annyhdrous ether, for example tetrahydrofuran, is preferable. With the other coupling agents the use of an acid scavenger, such as 4-dimethylaminopyridine or 4-pyrrolidinopyridine, is commonly preferred. With these coupling agents and catalysts a solvent such as anhydrous methylene chloride or chloroform may be used.

With the acid halide (preferably the acid chloride) a tertiary amine such as pyridine or triethylamine is preferred as an acid scavenger type catalyst and a solvent such as anhydrous methylene chloride or chloroform may be used.

In a preferred embodiment the acylation is carried out by reacting rapamycin with an acid of formula

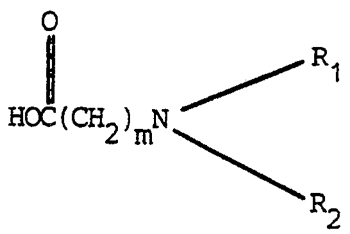

III

in the presence of a coupling agent, e.g. a carbodiimide (such as dicyclohexylcarbodiimide or diisopropylcarbodiimide) or carbonyldiimidazole.

A catalyst such as 4-dimethylaminopyridine or 4-pyrrolidinopyridine is preferred for use with such coupling agents. Coupling agents including carbodiimides and methods using them are well known in the art since they are extensively used in peptide chemistry - see for example E. Schroder and K. Lubke "The Peptides" Vol. 1, Acadamic Press, New York and London 1965 and Mr. Bodanszky & M.A.Ondetti, Peptide Synthesis 1966 Interscience USA.

Where one or both of $R_1$ and $R_2$ is hydrogen, protection of the amine function of the acylating agent by a protecting group which is removable under neutral conditions is preferred. For example, where one or both of $R_1$ and $R_2$ is hydrogen, the amine function of such acylating agent may be protected by using the acid chloride hydrochloride of the acylamino substituent as the acylating agent.

The acid chloride hydrochloride acylating agent may be made in a known manner by reacting the acid form of the acylating agent with one equivalent of hydrogen chloride gas then, with one equivalent phosphorous pentachloride. These reactions may be carried out in anhydrous methylene chloride. The product acid chloride hydrochloride may be recovered as a precipitate from toluene, hexane or cyclohexane. The acylation using said acid chloride hydrochloride may be carried out in a known manner using a weak base such as pyridine urea, dimethylaniline or trimethylamine as an acid scavenger.

The preparation of typical water soluble prodrugs of rapamycin of this invention is illustrated in the Examples which were carried out using the following procedures.

In the examples, chemical stability studies for rapamycin and the prodrugs were done at 20$\mu$g/ml with an ionic strength of 0.5. Stabilities at pH 3.3 (0.05 M acetate buffer) and pH 7.4 (0.05 M phosphate buffer) were studied at 25 and 37.5$^\circ$C. No antioxidants were added and the buffers were not deoxygenated.

The plasma studies were conducted at 37.5$^\circ$C for rat and human plasma. Rat plasma was obtained from Sprague-Dawley male albino rats and was used within several days. Human plasma was obtained from the Lawrence Memorial Hospital in Lawrence, Kansas. The plasma studies were done at three prodrug concentrations: 200, 100 and 50 $\mu$g/ml of prodrug. The experimental procedure was as follows: The compound to be tested was taken from a stock aqueous solution of 5mg/ml and added to the plasma to give the desired prodrug concentration. Samples of 200 $\mu$l were removed at predetermined times and added to 200 $\mu$l of 10% metaphosphoric acid to quench the reaction. Before centrifugation 200 $\mu$l of methanol was added to further precipitate the plasma proteins. The results are expressed in half-lives in hours.

The chemical and plasma studies were followed by HPLC using a RP C-18 column (150 mm) and a precolumn (50 mm). The mobile phase was 87:13 methanol: phosphate buffer (0.025 M, pH 3.4). The detector was set at 254 nm and the flow rate was 1 ml/min for rapamycin studies and 1.5 ml/min for rapamycin studies and 1.5 ml/min for the prodrug studies. Chart speed was 1 inch/10 minutes.

The liver homogenate studies were done using livers freshly obtained from male albino Sprague-Dawley rats. A 20% liver homogenate was prepared in Sorensen's buffer at pH 7.4. Chemical stability studies of rapamycin and the two prodrugs of Examples 2 and 3 were carried out at concentrations of 20, 50 and 50 $\mu$g/ml respectively, at 37.5C.

Rapamycin hydrolysis data in buffers, plasma and in rat liver homogenate are shown in the following table:

**TABLE 1**

Chemical Stability Study

| | | | |
|---|---|---|---|
| A. | 25° C | pH | $t_{1/2}$ (hrs) |
| | | 3.3 | 35.8 |
| | | 7.4 | 47.6 |
| B. | 37.5° C | pH | $t_{1/2}$ (hrs) |
| | | 3.3 | 9.9 |
| | | 7.4 | 10.2 |

Plasma Stability Study (37.5° C)

| | | | |
|---|---|---|---|
| A. | Human plasma | conc (µg/ml) | $t_{1/2}$ (hrs) |
| | | 50 | 3 |
| B. | Rat plasma | conc (µg/ml) | $t_{1/2}$ (hrs) |
| | | 50 | 2.83 |
| C. | Liver homogenate | conc (µg/ml) | $t_{1/2}$ (hrs) |
| | | 50 | 5.5 |

In all the prodrug studies, the disappearance of the prodrug peak appeared to result in the formation of a peak with a retention time nearly equal to rapamycin. Analysis of the plasma and homogenate studies by thin layer chromatography (TLC) tended to suggest that rapamycin initially formed but then it further degraded to other decomposition products, as does rapamycin itself in these studies.

This invention also provides an injectable pharmaceutical composition comprising a water-soluble derivative of rapamycin as defined above or a pharmaceutically acceptable salt thereof and a pharmaceutically acceptable carrier. Water or any water-based carrier known in art can be used, e.g. distilled water, water containing 5% by weight dextrose or a physiologically acceptable salt solution. Such physiologically acceptable salt solution should have a pH in the neutral range, as for example, normal saline or Lactate Ringers solutions.

## EXAMPLE 1

Synthesis of Mono-(28)-N,N-Dimethylglycinate Ester of Rapamycin

In a dry 100 mL round bottom flask was placed 2.80 g(3.07 X $10^{-3}$ moles) of rapamycin, 0.616 g (5.98 X $10^{-3}$ moles) of N,N-dimethyl glycine and 1.40 g (6.80 X $10^{-3}$ moles) of dicyclohexylcarbodiimide. The flask was placed under a nitrogen atmosphere and 60 mL of anhydrous methylene chloride (dried over $P_2O_5$) was added followed by 60 mg of 4-dimethylaminopyridine. The reaction was stirred overnight at room temperature. A thin layer chromatogram (TLC) of the reaction (solvent system 1:1 acetone:methylene chloride) was taken and indicated the reaction to be complete. The Rf of the monoglycinate prodrug was 0.32. Some bisglycinate was also present at a $R_f$ of 0.09. The reaction was worked-up by first filtering off the dicyclohexylurea (DCU). The solvent was removed on the rotovapor to give a white solid. The crude product was chromatographed on 18 gm of silica gel using 300 mL of ethyl acetate to elute rapamycin plus

residual DCU. The product was eluted with 1:1 methylene chloride:acetone to give 1.67 g of product, yield 55%. This material was found difficult to recrystallize. NMR (300 MHZ, solvent $CDCl_3$) indicated the spectrum of the prodrug to be practically identical to that of rapamycin except for the two singlets arising from the glycinate group. The N,N dimethyl protons appeared as a singlet at δ2.32. The methylene group of the glycinate was found at δ3.16 as a singlet.

## EXAMPLE 2

Synthesis of Methanesulfonic Acid Salt of Mono-(28)-N,N Dimethylglycinate Ester of Rapamycin

In a dry 100 mL round bottom flask was placed 3.00 g ($3.10 \times 10^{-3}$ moles) of mono N,N-dimethylglycinate prodrug of rapamycin. This was dissolved in 15 mL of anhydrous methylene chloride (distilled from $P_2O_5$). To this was added $2.71 \times 10^{-3}$ moles) of a stock solution of methanesulfonic acid dissolved in diethyl ether. The solvent was immediately removed to give a white solid, wt. 3.25 g, yield 99%. This compound was also found difficult to recrystallize. The salt form of this compound was found to be unstable to long stirring times. Even in the crystalline form long exposures to light resulted in a slow discoloration of the material.

Data with respect to mono-(28)-N,N-dimethylglycinate methanesulfonicc acid salt-prodrug of rapamycin are shown in the following table:

### TABLE 2

**Physical Properties**

| | |
|---|---|
| MW | 1095 |
| MP | 93-99°C |
| Solubility in water | > 50 mg/mL |

**HPLC Operating Conditions**

| | |
|---|---|
| Column | RP-18, 150 mm length, 4.6 mm id |
| Precolumn | 50 mm length, 4.6 mm id |
| Mobile phase | 87 parts methanol:13 parts phosphate buffer (0.025 M, pH 3.4) |
| Detector | Kratos 783<br>UV 254 nm |
| Flow rate | 1.5 mL/min |
| Retention | 9.5 mL* |

* With a new RP C-18 column two peaks were observed which are believed to be cis-trans isomers about the amide bond in the macrocyclic lactone ring.

**Chemical Stability, 25° C**

| Conditions | $t_{1/2}$(hrs) |
|---|---|
| pH 3.3 | 73 |
| pH 7.4 | 45 |

**Plasma/Tissue Stability, 37.5° C**

| Conditions | $t_{1/2}$ (hrs) |
|---|---|
| 50 µg prodrug/mL human plasma | 5 |
| 50 µg prodrug/mL rat plasma | 1.8 |
| 50 µg prodrug/mL liver homogenate | 4.5 |

Plasma/Tissue Stability Study (37.5 C)

| | | | |
|---|---|---|---|
| A. | Human plasma | conc (µg/ml) | $t_{1/2}$ (hrs) |
| | | 200 | 5.6 |
| | | 100 | 4.8 |
| | | 50 | 5.0 |
| B. | Rat plasma | conc (µg/ml) | $t_{1/2}$ (hrs) |
| | | 200 | 2.5 |
| | | 100 | 1.8 |
| | | 50 | 1.75 |
| C. | Liver homogenate | conc (µg/ml) | $t_{1/2}$ (hrs) |
| | | 50 | 4.5 |

**Reconstitution Procedure**

The prodrug can be reconstituted with either water for injection or distilled water containing 5% by weight dextrose (D5W). The solutions should be freshly prepared and used immediately (<1 hr if possible). The prodrug appears to discolor upon prolonged exposure to light. Precaution should be taken to prevent this.

EXAMPLE 3

Synthesis of Mono-(28)-3-(N,N-Diethylamino)propionate Hydrochloride Salt Ester of Rapamycin

In a dry 100 mL round bottom flask was placed 1.00 g (1.09 X $10^{-3}$ moles) of rapamycin, 0.34 g (2.16 X $10^{-3}$ moles) N,N-diethylaminopropionic acid hydrochloride salt and 0.50 g (2.43 X $10^{-3}$ moles) of dicyclohexylcarbodiimide.

The vessel was placed under a nitrogen atmosphere and 25 mL of anhydrous methylene chloride (dried over $P_2O_5$) was added followed by 15 mg of 4-dimethylaminopyridine. The reaction was stirred overnight at room temperature. The next day a TLC of the reaction (solvent system: ethyl acetate) on silanized silica gel plate was taken and indicated the reaction to be complete. The $R_f$ of the monopropionate hydrochloride salt of rapamycin was 0.34 and 0.01 for the bispropionate hydrochloride salt which was also formed in the reaction. The dicyclohexylurea was filtered from the reaction and the solvent removed on the rotovapor. The crude product was chromatographed on 12 g of silanized silica gel. The column was first developed with 200 mL of ethyl acetate to remove any rapamycin and also residual dicyclohexylurea. The product was eluted with ethyl acetate to give 0.61 g of product, yield 53%. This compound was found difficult to recrystallize and unstable to prolonged exposure to light. NMR (300 MHz, solvent $CDCL_3$) indicated the spectrum of the prodrug to be practically identical with that of rapamycin. The propionate group did not give sharp easily interpreted resonances as was the case with the glycinate prodrug. This is the result of the resonances being multiplets resulting from the ethyl groups which are not as easily seen among the other resonances from rapamycin. Broad peaks did appear around $\delta 1.2$ and $\delta 1.5$ which were not found in rapamycin.

Data with respect to mono-(28)-N,N-diethylaminopropionate hydrochloride salt - prodrug of rapamycin are shown in the following table:

## TABLE 3

**Physical Properties**

| | |
|---|---|
| M.W. | 1077 |
| M.P. | 99-106°C |
| Solubility | >50 mg/mL in water |

**HPLC Operating Conditions**

| | |
|---|---|
| Column | RP - 18, 150 mm length, 4.6 mm id |
| Precolumn | 50 mm length, 4.6 mm id |
| Mobile phase | 87 parts methanol: 13 parts phosphate buffer (0.025 M, pH 3.4) |
| Detector | Kratos 783<br>UV 254 nm |
| Flow rate | 1.5 mL/min |
| Retention volume | 9.75 mL* |

*Two peaks were also observed for this prodrug when a new RP-18 column was used. This was also believed to be cis-trans isomers as mentioned above for the glycinate prodrug.

**Chemical Stability**

| Conditions | $t_{1/2}$(hrs) |
|---|---|
| pH 3.3,25°C | 33 |
| pH 7.4,25°C | 17 |
| pH 3.3, 37.5°C | 7.9 |
| pH 7.4, 37.5°C | 6.3 |

**Plasma/Tissue Stability, 37.5° C**

| Conditions | $t_{1/2}$(hrs) |
|---|---|
| 50 µg prodrug/mL human plasma | 2.5 |
| 50 µg prodrug/mL rat plasma | 1 |
| 50 µg prodrug/mL liver homogenate | 3.7 |

Plasma/Tissue Stability Study (37.5° C)

| | | | |
|---|---|---|---|
| A. | Human plasma | conc (µg/ml) | $t_{1/2}$ (hrs) |
| | | 200 | 3.25 |
| | | 100 | 2.15 |
| | | 50 | 2.50 |
| B. | Rat plasma | conc (µg/ml) | $t_{1/2}$ (min) |
| | | 200 | 60 |
| | | 100 | 58 |
| | | 50 | 58 |
| C. | Liver homogenate | conc (µg/ml) | $t_{1/2}$ (hrs) |
| | | 50 | 3.7 |

**Reconstitution Procedure**

The prodrug can be reconstituted with either water for injection or D5W. The solutions should be freshly prepared and used immediately (<1 hr if possible). The prodrug appears to discolor upon prolonged exposure to light. Precaution should be taken to prevent this.

**EXAMPLE 4**

Synthesis of Mono-(28)-4'-(N-pyrrolidino)-butyrate Hydrochloride Salt Ester of Rapamycin.

In a dry 100 mL round bottom flask was placed 3.50 g ($3.83 \times 10^{-3}$ moles) of rapamycin, 1.48 g ($7.66 \times 10^{-3}$ moles) of 4-pyrrolidino-butyric acid hydrochloride salt and 50 mL of anhydrous methylene chloride (distilled from $P_2O_5$). The reaction was placed under a nitrogen atmosphere and 2.50 g ($1.21 \times 10^{-2}$ moles) of dicyclohexylcarbodiimide and 15 mg of 4-N,N-dimethylaminopyridine. The reaction was stirred overnight at room temperature. The following day the dicyclohexylurea was filtered from the reaction and the filtrate adsorbed onto 5 g of silanized silica gel. This was loaded onto a 12 g column of silanized silica gel and was developed with 75:25 ethyl acetate: hexane to remove the starting material. The product was eluted with ethylacetate to give 3.24 g of a white solid, yield 78%.

Data with respect to the mono-(28)-4'-(pyrrolidino)butyrate hydrochloride salt-prodrug of rapamycin are shown below:

**Physical Properties**

| | |
|---|---|
| M.W. | 1088 |
| M.P. | 94-98° C |
| Solubility | ~15 mg/mL in water |

Reconstitution Procedure

The prodrug can be reconstituted with either water for injection or D5W. The solutions should be freshly prepared and used immediately (<1 hr if possible). The prodrug appears to discolor upon prolonged exposure to light. Precaution should be taken to prevent this.

**EXAMPLE 5**

Synthesis of Bis N,N-Dimethylglycinate Ester of Rapamycin

a) The bis-glycinate prodrug of rapamycin substituted at positions 28 and 43 of the rapamycin structure was synthesized by the addition of 1 eq. of rapamycin, 3 eq. of N,N-dimethylglycine, 3.3 eq. of dicyclohexylcarbodiimide and 0.16 eq. of 4-N,N-dimethylaminopyridine. After purification on silica gel, 64% of bis-glycinate was obtained. NMR confirmed the product with two 6 proton singlets for the methyl groups of the two glycinate groups.

The formation of the methane sulfonic acid salt of the bis-glycinate was accomplished by the addition of 1.95 eq. of methane sulfonic acid. The use of two equivalents caused the decomposition of the prodrug. This gave 92% yield of the bis-glycinate prodrug of rapamycin.

The studies carried out using fresh human plasma and fresh rat plasma indicate that the half life of the prodrug of Example 3 was the shortest, i.e. that half of the prodrug decomposed into products including mainly rapamycin within two and one-half hours with rapamycin being the only observed product of hydrolysis.

b) Similarly as in Example 1, other water soluble derivatives of rapamycin can be prepared using as a reagent instead of N,N-dimethyl glycine, glycine, N,N-diethylglycine, N,N-diisopropylglycine, N-propylglycine, 3-aminopropionic acid, N-ethyl-3-aminopropionic acid, 4-aminobutyric acid, N-ethyl-4-amino butyric acid, N,N-dipropyl-4-aminobutyric acid, 2-(N-pyrrolidino) acetic acid, and 3-(N-piperidino) propionic acid and using appropriate protecting groups where necessary.

## Claims

**1.** A water-soluble pharmaceutically acceptable salt of a mono-acyl derivative of rapamycin, said salt being obtainable as the major monoacyl product of the reaction of rapamycin with an acid of formula

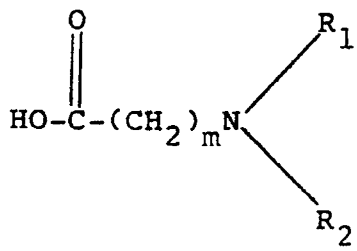

or an acid addition salt thereof,
wherein m is an integer from 1 to 3, and $R_1$ and $R_2$ are each hydrogen or an alkyl radical having from one to three carbon atoms or wherein $R_1$ and $R_2$ together with the nitrogen atom to which they are attached to form a saturated heterocyclic ring having four to five carbon atoms, in the presence of a coupling agent, followed if necessary by acidification to give the pharmaceutically acceptable salt thereof.

2. A rapamycin derivative as claimed in Claim 1 wherein $R_1$ and $R_2$ are each an alkyl radical of 1 to 3 carbon atoms.

3. A rapamycin derivative as claimed in Claim 2 wherein $R_1$ and $R_2$ are methyl or ethyl.

4. A rapamycin derivative as claimed in any one of Claims 1 to 3 wherein m is 1 or 2.

5. The rapamycin derivative of Claim 1 wherein m is 1 and $R_1$ and $R_2$ are both methyl.

6. The rapamycin derivative of Claim 1 wherein m is 2 and $R_1$ and $R_2$ are both ethyl.

7. The rapamycin derivative of Claim 1 wherein m is 3 and $R_1$ and $R_2$ together with the nitrogen atom to which they are attached represents

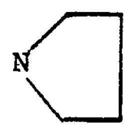

8. A process for preparing a water-soluble pharmaceutically acceptable salt of a monoacyl derivative of rapamycin as claimed in Claim 1 which comprises reacting rapamycin with an acylating agent containing the acylamino substituent having the configuration

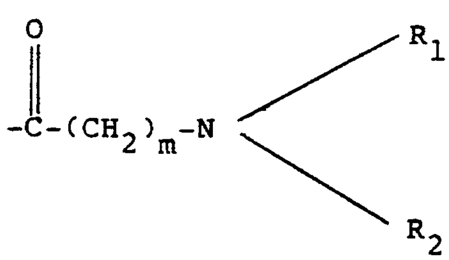

wherein m, $R_1$ and $R_2$ are as defined in Claim 1, and if required acidifying to give a pharmaceutically acceptable salt thereof.

9. A process as claimed in Claim 8 wherein the acylating agent is the acid, the acid halide, the acid anhydride or an activated ester of said acylamino substituent.

10. A process as claimed in Claim 9 wherein the acylating agent is the acid of said acylamino substituent in the presence of a coupling agent.

11. A process as claimed in Claim 10 wherein the coupling agent is N, N'-dicyclohexylcarbodiimide, 1,1'-carbonyldiimidazole, diethylazodicarboxylate, 2,2'-dithiopyridine, or N,N-diisopropylcarbodiimide.

12. A process as claimed in any one of Claims 8 to 11 wherein $R_1$ and $R_2$ are each alkyl of 1 to 3 carbon atoms.

13. An injectable pharmaceutical composition comprising a water soluble monoacyl derivative of rapamycin as claimed in any one of Claims 1 to 8 and a pharmaceutically acceptable carrier.

CLAIMS FOR THE FOLLOWING CONTRACTING STATES: AT, GR, ES

1. A process for preparing a water soluble pharmaceutically acceptable salt of a monoacyl derivative of rapamycin which comprises acylating rapamycin with an acylating agent containing an acylamino substituent having the configuration:

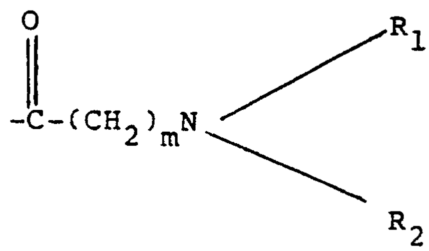

wherein m is an integer from 1 to 3,
wherein $R_1$ and $R_2$ are each hydrogen or an alkyl radical having from one to three carbon atoms or wherein $R_1$ $R_2$ together with the nitrogen to which they are attached to form a saturated heterocyclic ring having four to five carbon atoms and isolating the major monoacyl product, if required acidifying the product to give a pharmaceutically acceptable salt thereof.

2. A process as claimed in Claim 1 wherein $R_1$ and $R_2$ are each an alkyl radical of 1 to 3 carbon atoms.

3. A process as claimed in Claim 2 wherein $R_1$ and $R_2$ are methyl or ethyl.

4. A process as claimed in any one of Claims 1 to 3 wherein m is 1 or 2.

5. The process as claimed in Claim 1 in which the mono-substituted derivative is prepared having the substituent

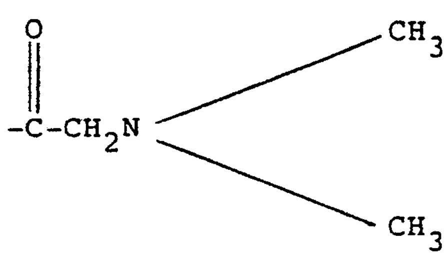

6. A process as claimed in Claim 1 in which the mono substituted derivative is prepared having the substituent

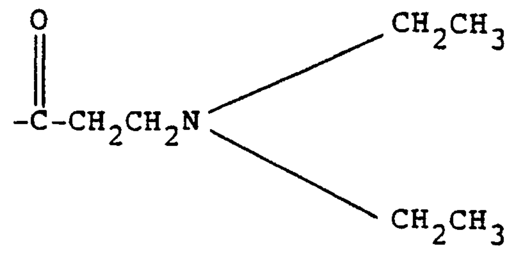

7. A process as claimed in Claim 1 in which the mono substituted derivative is prepared having the substituent

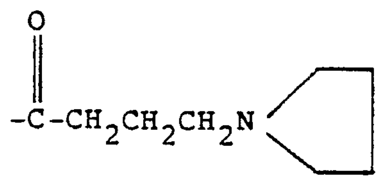

8. A process as claimed in Claim 1 wherein the acylating agent is the acid, the acid halide, the acid anhydride or an activated ester of said acylamino substituent.

9. A process as claimed in Claim 1 wherein the acylating agent is the acid of said acylamino substituent in the presence of a coupling agent.

10. A process as claimed in Claim 9 wherein the coupling agent is N, N'-dicyclohexylcarbodiimide, 1,1'-carbonyldiimidazole, diethylazodicarboxylate, 2,2'-dithiopyridine, or N,N-diisopropylcarbodiimide.

11. A process for preparing an injectable pharmaceutical composition which comprises bringing a water soluble derivative of rapamycin or a pharmaceutically acceptable salt thereof as defined in any one of Claims 1 to 7 into a form suitable for therapeutic administration.